# EUROPEAN PATENT APPLICATION

(11) **EP 4 335 482 A1**
(43) Date of publication of application: **13.03.2024**
(21) Application number: 21840417.6
(22) Date of filing: 25.10.2021
(51) Int. Cl.: A61M 37/00, A61K 9/00

(54) **MICRONEEDLE PATCH**

(30) Priority: 04.05.2021 KR 20210057937
(71) Applicant: Feroka Inc., Seoul 04784 (KR)
(72) Inventor: LEE, Jae Joon, Seoul 02793 (KR); JEON, Yi Seul, Seoul 08799 (KR)
(74) Representative: Hoffmann Eitle
(86) International application number: PCT/KR2021/015024
(87) International publication number: WO 2022/234905

(57) **Abstract**

A microneedle patch includes: a base; and a microneedle, which protrudes from a surface of the base, includes a plurality of layers, and has curvatures in a region where the layers are in contact with each other.

## Description

### TECHNICAL FIELD

The present disclosure relates to a microneedle patch.

### BACKGROUND

Injection of a drug into a human body has traditionally been performed by using a needle syringe, but such needle-syringe injection causes great pain. Noninvasive drug injection methods have been developed to overcome this issue, but these methods have a problem in that a large amount of drug is consumed compared to the amount of actually delivered drug.

In order to find a solution to this problem, many studies have been conducted on drug delivery systems (DDSs), and these studies have made even greater advances with the development of nanotechnology.

Unlike conventional injection needles, microneedles enable painless skin penetration without injury. In addition, a certain degree of physical hardness of microneedles may be required to penetrate the stratum corneum of skin. In addition, an appropriate length of microneedles may be required for physiologically active substances to reach the epidermal or dermal layer of skin. Furthermore, in order to effectively deliver physiologically active substances in hundreds of microneedles into skin, the microneedles need to have high skin permeability and be maintained for a certain period of time until dissolution after being inserted into the skin.

Accordingly, interest in microneedles capable of delivering a precise amount of a drug and accurately setting a target position is increasing.

### DESCRIPTION OF EMBODIMENTS

### TECHNICAL PROBLEM

The present disclosure may provide a microneedle patch capable of effectively delivering a preset amount of an effective ingredient to a target position.

### TECHNICAL SOLUTION TO PROBLEM

An embodiment of the present disclosure provides a microneedle patch including a base, and a microneedle, which protrudes from the surface of the base, includes a plurality of layers, and has a curvature in a region at which the layers are in contact with each other.

### ADVANTAGEOUS EFFECTS OF PRESENT DISCLOSURE

A microneedle patch according to the present disclosure has a multi-layer structure, and thus is capable of accurately delivering an effective ingredient to a target point. The microneedle includes a plurality of layers, and thus an effective ingredient may be arranged in each layer. Accordingly, the microneedle patch may deliver the effective ingredients to any one of an epidermis, a dermis, subcutaneous fat, and muscle, according to positions at which the effective ingredients are activated, respectively.

The microneedle patch according to the present disclosure has a multi-layer structure, and thus the biodegradation rates of the layers may be different from each other. The effective ingredients of the layers of the microneedle may be activated at different points of time according to the decomposition rates of the layers.

In the microneedle patch according to the present disclosure, each layer has a curvature, and thus may easily decompose in vivo. As the first layer and the second layer have the curvatures, the distance between a region at which the first layer and the second layer are in contact with each other and the edge of the microneedle is short, and thus the first layer and the second layer may be easily separated from each other.

In the microneedle patch according to the present disclosure, because each layer has a curvature, the surface area of each layer is increased, and accordingly, the delivery effectiveness of an effective ingredient may be increased. The first curved surface of the first layer increases the upper surface area of the first layer, and the second curved surface of the second layer increases the lower surface area of the second layer. As the surface areas of the first layer and the second layer are increased, an area through which a drug is delivered may be also increased, and thus, the drug delivery effectiveness may be improved.

### BRIEF DESCRIPTION OF DRAWINGS

FIG. 1 is a perspective view illustrating a microneedle patch according to an embodiment of the present disclosure.
FIG. 2 is a cross-sectional view illustrating the microneedle patch of FIG. 1.
FIG. 3 is an enlarged view of a portion of FIG. 2.
FIG. 4 is an enlarged view of region A of FIG. 3.
FIG. 5 is a diagram illustrating a process in which the microneedle patch of FIG. 2 is placed on a skin, and then a drug is delivered.
FIG. 6 to FIG. 10 are diagrams illustrating modifications of the microneedle of FIG. 3.
FIG. 11 is a diagram illustrating a microneedle patch according to another embodiment of the present disclosure.
FIG. 12 is a diagram illustrating a process in which the microneedle patch of FIG. 11 is placed on a skin, and then a drug is delivered.
FIG. 13 to FIG. 18 are diagrams illustrating modifications of the microneedle patch of FIG. 11.
FIG. 19 is a diagram illustrating a microneedle patch according to another embodiment of the present disclosure.
FIG. 20 is a diagram illustrating a modification of the microneedle patch of FIG. 19.
FIG. 21 is a diagram illustrating a microneedle patch according to another embodiment of the present disclosure.
FIG. 22 is a diagram illustrating a modification of the microneedle patch of FIG. 21.
FIG. 23 is a flowchart of a method of manufacturing a microneedle patch, according to another embodiment of the present disclosure.

### MODE OF DISCLOSURE

An embodiment of the present disclosure provides a microneedle patch including a base, and a microneedle, which protrudes from the surface of the base, includes a plurality of layers, and has a curvature in a region where the layers are in contact with each other.

In addition, the microneedle may include: a first layer having a sharpened tip at one side thereof and a first curved surface at the other side thereof, the first curved surface being recessed toward the sharpened tip; and a second layer arranged to face the first curved surface and having a second curved surface protruding to correspond to the first curved surface.

In addition, the second layer may have a third curved surface arranged to be opposite to the second curved surface and recessed toward the second curved surface.

In addition, the curvature of the first curved surface and a curvature of the third curved surface may be different from each other.

In addition, at least one of the first curved surface and the second curved surface may have a plurality of curvatures.

In addition, both ends of the curvature of the first curved surface may be symmetrical to each other with respect to a longitudinal direction of the microneedle.

In addition, in the microneedle, a roughness of a region where adjacent layers are in contact with each other may be different from a roughness of an outer surface of the microneedle.

In addition, the microneedle may further include an adhesive layer arranged between the adjacent layers.

In addition, at least one of the plurality of layers of the microneedle may include an in vivo degradable polymer.

Another embodiment of the present disclosure provides a microneedle patch including: a base; and a microneedle protruding from a surface of the base and including a plurality of layers, wherein a roughness of a region where adjacent layers are in contact with each other is different from a roughness of an outer surface of the microneedle.

Other aspects, features, and advantages other than those described above will be apparent from the following drawings, claims, and detailed description.

### DETAILED DESCRIPTION

Hereinafter, features and functions of the present disclosure will be described in detail with reference to embodiments illustrated in the accompanying drawings.

As the present disclosure allows for various changes and numerous embodiments, particular embodiments will be illustrated in the drawings and described in detail. The effects and features of the present disclosure and methods of achieving them will become clear with reference to the embodiments described in detail below with the drawings. However, the present disclosure is not limited to the embodiments disclosed below, and may be implemented in various forms.

Hereinafter, embodiments of the present disclosure will be described in detail with reference to the accompanying drawings, and the same or corresponding components will be denoted by the same reference numerals when described with reference to the accompanying drawings, and thus their descriptions that are already provided will be omitted.

While such terms as "first," "second," etc., are used only to distinguish one component from another, and such components must not be limited by these terms.

The singular expression also includes the plural meaning as long as it is not inconsistent with the context.

The terms "comprises," "includes," or "has" used herein specify the presence of stated features or components, but do not preclude the presence or addition of one or more other features or components.

For ease of description, the magnitude of components in the drawings may be exaggerated or reduced. For example, the magnitude and thickness of each component in the drawings is illustrated for ease of description, and the present disclosure is not limited to the drawings.

When a certain embodiment may be differently implemented, a specific process sequence may be performed differently from the sequence described herein. For example, two processes, which are successively described herein, may be substantially simultaneously performed, or may be performed in a process sequence opposite to a described process sequence.

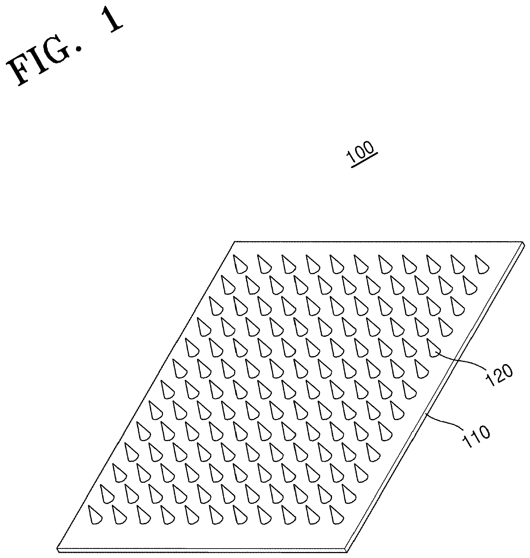

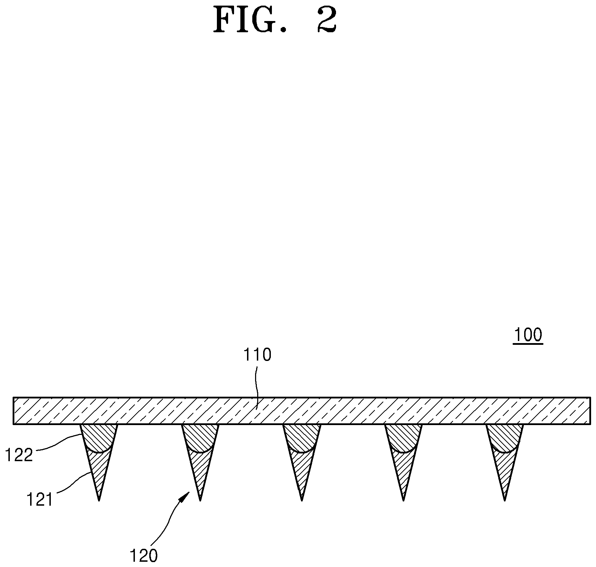

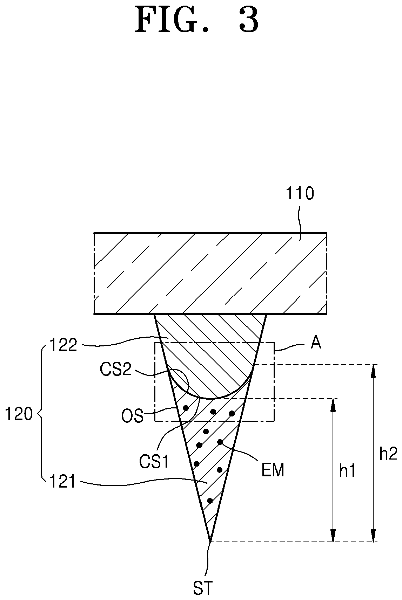

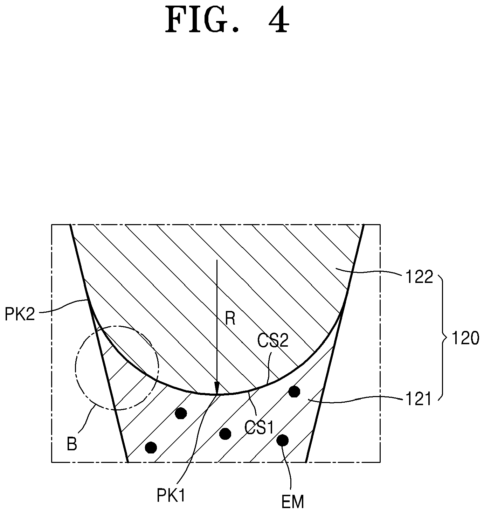

FIG. 1 is a perspective view illustrating a microneedle patch 100 according to an embodiment of the present disclosure, FIG. 2 is a cross-sectional view illustrating the microneedle patch 120 of FIG. 1, FIG. 3 is an enlarged view of a portion of FIG. 2, and FIG. 4 is an enlarged view of region A of FIG. 3.

Referring to FIGS. 1 to 4, the microneedle patch 100 may include a base 110 and the microneedles 120.

The plurality of microneedles 120 may be provided on one surface of the base 110, and may be supported by the base 110. The one surface of the base 110 may come into contact with skin, and the other surface of the base 110 may be exposed to the outside.

The base 110 may be removed after the microneedles 120 are inserted into the skin. For example, the base 110 may be removed from the skin by a user applying a force. As another example, a portion at which the base 110 and the microneedle 120 are coupled to each other first dissolves, and thus the base 110 may be removed after a certain period of time has elapsed after the microneedle patch 100 is attached to the skin. As yet another example, the base 110 may dissolve after a long period of time has elapsed after the microneedle patch 100 is attached to the skin. As yet another example, the base 110 may be removed by the user coating a material for dissolution.

In an embodiment, the base 110 may include any one of materials included in the microneedle 120. The base 110 may include a biodegradable material similarly to the microneedle 120. For example, the base 110 may include the same material as that of any one of a plurality of layers of the microneedle 120.

In an alternative embodiment, the base 110 may include a physiologically active substance. After attaching the microneedle patch 100 to the skin, an effective drug may be effectively delivered to a patient by the physiologically active substance released from the base 110. In addition, the base 110 and the microneedles 120 may be easily separated from each other by the physiologically active substance released from the base 110.

In an embodiment, the base 110 may have a property of dissolving later than does the closest layer of the microneedle 120, i.e., a layer that is farthest away from the tip of the microneedle 120. Because a portion of the microneedle 120, which is adjacent to the base 110, dissolves the fastest, the base 110 may be easily separated from the microneedle 120.

In an embodiment, the base 110 may include a water-soluble polymer. The base 110 may be formed of a water-soluble polymer or may include other additives (e.g., disaccharides, etc.). In addition, it is preferable that the base 110 does not include a drug or an effective ingredient.

The base 110 may include a biocompatible material. A biocompatible material selected as a base material of the microneedle 120, which will be described below, may also be selected as a base material of the base 110.

The plurality of microneedles 120 may be formed to protrude from the surface of the base 110. The microneedle 120 may be formed of a biocompatible material and an additive.

The biocompatible material may include at least any one of carboxymethyl cellulose (CMC), hyaluronic acid (HA), alginic acid, pectin, carrageenan, chondroitin sulfate, dextran sulfate, chitosan, polylysine, carboxymethyl chitin, fibrin, agarose, pullulan, polyanhydride, polyorthoester, polyetherester, polyesteramide, polybutyric acid, polyvaleric acid, polyacrylate, an ethylene-vinyl acetate polymer, acrylsubstituted cellulose acetate, polyvinyl chloride, polyvinyl fluoride, polyvinyl imidazole, chlorosulphonate polyolefins, polyethylene oxide, polyvinylpyrrolidone (PVP), hydroxypropyl methylcellulose (HPMC), ethylcellulose (EC), hydroxypropyl cellulose (HPC), cyclodextrin, maltose, lactose, trehalose, cellobiose, isomaltose, turanose, and lactulose, or at least any one of a copolymer of monomers forming such polymers and cellulose.

The additive may include at least any one of trehalose, oligosaccharide, sucrose, maltose, lactose, cellobiose, HA, alginic acid, pectin, carrageenan, chondroitin sulfate, dextran sulfate, chitosan, polylysine, collagen, gelatin, carboxymethyl chitin, fibrin, agarose, PVP, polyethylene glycol (PEG), polymethacrylate, HPMC, EC, HPC, carboxymethyl cellulose, cyclodextrin, gentiobiose, cetrimide (alkyltrimethylammonium bromide), cetrimonium bromide (hexadecyltrimethylammonium bromide (CTAB)), gentian violet, benzethonium chloride, docusate sodium salt, a SPAN-type surfactant, polysorbate (Tween), sodium lauryl sulfate (sodium dodecyl sulfate (SDS)), benzalkonium chloride, and glyceryl oleate.

The term "hyaluronic acid (HA)" is used herein to encompass hyaluronic acid, hyaluronates (e.g., sodium hyaluronate, potassium hyaluronate, magnesium hyaluronate, and calcium hyaluronate), and mixtures thereof. The term "hyaluronic acid (HA)" is also used here to encompass cross-linked hyaluronic acid and/or noncross-linked hyaluronic acid.

According to an embodiment of the present disclosure, the molecular weight of the HA is 2 kDa to 5000 kDa.

According to another embodiment of the present disclosure, the molecular weight of the HA is 100 kDa to 4500 kDa, 150 kDa to 3500 kDa, 200 kDa to 2500 kDa, 220 kDa to 1500 kDa, 240 kDa to 1000 kDa, or 240 kDa to 490 kDa.

The CMC used herein may be known CMC with various molecular weights. For example, the average molecular weight of the CMC used herein is 90,000 kDa, 250,000 kDa, or 700,000 kDa.

The disaccharides may be sucrose, lactulose, lactose, maltose, trehalose, cellobiose, or the like, and may particularly include sucrose, maltose, and trehalose.

In an alternative embodiment, the microneedle 120 may include an adhesive. The adhesive is at least one adhesive selected from the group consisting of silicone, polyurethane, HA, a physical adhesive (Gecko), polyacryl, EC, hydroxymethyl cellulose, ethylene-vinyl acetate, and polyisobutylene.

In an alternative embodiment, the microneedle 120 may further include a metal, a polymer, or an adhesive.

The microneedle 120 may include an effective ingredient EM. The microneedle 120 may include the effective ingredient EM in at least a portion thereof, and the effective ingredient EM may be a pharmaceutically, medically, or cosmetically effective ingredient. For example, the effective ingredient includes, but is not limited to, a protein/peptide medicine, and may include at least one of a hormone, a hormone analogue, an enzyme, an enzyme inhibitor, a signal transduction protein or a portion thereof, an antibody or a portion thereof, a singlechain antibody, a binding protein or a binding domain thereof, an antigen, an adherent protein, a structural protein, a regulatory protein, a toxic protein, a cytokine, a transcription regulator, a blood coagulation factor, and a vaccine. In detail, the protein/peptide medicine may include at least one of insulin, insulin-like growth factor 1 (IGF-1), growth hormone, erythropoietin, granulocyte colony-stimulating factors (G-CSFs), granulocyte/macrophage colony-stimulating factors (GM-CSFs), interferon alpha, interferon beta, interferon gamma, interleukin-1 alpha and beta, interleukin-3, interleukin-4, interleukin-6, interleukin-2, epidermal growth factors (EGFs), calcitonin, adrenocorticotropic hormone (ACTH), tumor necrosis factor (TNF), atobisban, buserelin, cetrorelix, deslorelin, desmopressin, dynorphin A (1-13), elcatonin, eleidosin, eptifibatide, growth hormone releasing hormone-II (GHRH-II), gonadorelin, goserelin, histrelin, leuprorelin, lypressin, octreotide, oxytocin, pitressin, secretin, sincalide, terlipressin, thymopentin, thymosine, triptorelin, bivalirudin, carbetocin, cyclosporine, exedine, lanreotide, luteinizing hormone-releasing hormone (LHRH), nafarelin, parathyroid hormone, pramlintide, enfuvirtide (T-20), thymalfasin, and ziconotide. In addition, the effective ingredient EM may be a cosmetic ingredient such as a skin lightening agent, a filler, a wrinkle reducing agent, or an antioxidant.

In an embodiment, the effective ingredient EM may be colloid particles dispersed in a solvent forming the microneedle 120. The particles themselves may be the effective ingredient EM or may include a coating material carrying the effective ingredient EM.

The effective ingredient EM may be intensively distributed in a partial layer of the microneedle 120. That is, the effective ingredient EM may be at a certain height in the microneedle 120, and thus, the effective ingredient EM may be effectively delivered.

In another embodiment, the effective ingredient EM may be dissolved in the microneedle 120. The effective ingredient EM may be dissolved in the base material of the microneedle 120, such as the biodegradable materials described above, to constitute the microneedle 120. The effective ingredient EM may be uniformly dissolved in the base material and may be intensively distributed at a certain height of the microneedle 120, like the above-described particles.

In an embodiment, the microneedle patch 100 may have a plurality of effective ingredients EM in respective regions thereof. A first group of microneedles among the plurality of microneedles may include a first effective ingredient among the plurality of effective ingredients, and a second group of microneedles different from the first group may include a second effective ingredient among the plurality of effective ingredients.

In an embodiment, the pharmaceutically, medically, or cosmetically effective ingredient EM may be coated on the microneedle 120. The effective ingredients EM may be coated on the entire microneedle 120 or only a portion of the microneedle 120. Alternatively, in the microneedle 120, the first effective ingredient may be coated on a portion of a coating layer, and the second effective ingredient may be coated on another portion of the coating layer.

The microneedle 120 may have various shapes. The microneedle 120 may have a conical shape. For example, the microneedle 120 may have a polygonal shape such as a conical shape, a triangular pyramid shape, or a quadrangular pyramid shape.

The microneedle 120 may have a layered structure. The microneedle 120 may have a plurality of stacked layers. The number of layers constituting the microneedle 120 is not limited to a certain number. However, for convenience of description, the microneedle 120 having two layers will be mainly described.

The microneedle 120 may include a first layer 121 arranged at an end thereof, and a second layer 122 stacked on the first layer 121. The microneedle 120 may have a curvature in a region where adjacent layers are in contact with each other. That is, a curvature may be formed in a region where the first layer 121 and the second layer 122 are in contact with each other.

The microneedle 120 may have a curvature downwardly convex toward the tip. In the region where the first layer 121 and the second layer 122 are in contact with each other, a portion adjacent to the tip may have a downwardly convex shape. The curvature may be formed to have both sides symmetrical to each other with respect to the longitudinal direction of the microneedle 120.

The first layer 121 may have a sharpened tip ST arranged at one side thereof, and a first curved surface CS1 recessed toward the sharpened tip ST at the other side thereof.

The first layer 121 may have a first point PK1 at the center of the microneedle 120 in the longitudinal direction, and a first height h1 between the sharpened tip ST and the first point PK1. The first layer 121 may have a second point PK2 at an outer side in the radial direction, and a second height h2 between the sharpened tip ST and the second point PK2. The first height h1 may be less than the second height h2.

In an embodiment, the height of the first layer 121, i.e., the distance between the tip ST and the first layer 121, may increase in the direction from the center in the longitudinal direction of the microneedle 120 to the outer periphery in the radial direction.

In an embodiment, the first layer 121 may have a curvature, such that the height of the first layer 121 varies in the direction from the center in the longitudinal direction of the microneedle 120 to the outer periphery. For example, the height of the first layer 121 may increase in the direction from the center to the outer periphery of the first layer 121.

In another embodiment, the height of the first layer 121 may linearly vary. Although not illustrated in the drawings, the height of the first layer 121 may linearly vary in the direction from the first point PK1 to the second point PK2.

The second layer 122 may face the first curved surface CS1, and may have a second curved surface CS2 protruding to correspond to the first curved surface CS1. The second curved surface CS2 may be connected to the first curved surface CS1, and thus, the first curved surface CS1 and the second curved surface CS2 may form a contact region. Accordingly, the curvature of the second curved surface CS2 may be substantially the same as the curvature of the first curved surface CS1.

Referring to FIG. 4, the first layer 121 and the second layer 122 may have a curvature R in the boundary region therebetween. The first curved surface CS1 and the second curved surface CS2 may have the same radius of curvature across the entire boundary region.

Because the microneedle 120 has the layered structure with the curvature, each layer may be easily separated. The contact portion of the first layer 121 and the second layer 122 is thin at the edge of the microneedle 120. In detail, in FIG. 4, the distance between an outer surface OS of the first layer 121 and the first curved surface CS1 is relatively short. When the microneedle 120 is inserted into the skin, the outer surface OS begins to biodegrade, and because the first layer 121 is easily separated by the first curved surface CS1, the first layer 121 and the second layer 122 may be easily separated from each other.

At least one of the first layer 121 and the second layer 122 of the microneedle 120 may include the effective ingredient EM. Depending on a target position of the effective ingredient EM, at least one of the plurality of layers of the microneedle 120 may include the effective ingredient EM.

In an embodiment, as illustrated in FIG. 3, the effective ingredient EM may be arranged in the first layer 121. In order to target a drug to a position slightly deep from a skin surface, the effective ingredient EM may be included in the first layer 121. For example, in order to deliver the effective ingredient EM to a dermis DEM, a subcutaneous fat layer, or muscle, the effective ingredient EM may be included in the first layer 121.

In another embodiment, although not illustrated in the drawings, the effective ingredient EM may be arranged in the second layer 122. In order to deliver a drug to a portion relatively shallow from the skin surface, the effective ingredient EM may be included in the second layer 122. For example, in order to deliver the effective ingredient EM to an epidermis EPM or a portion of the dermis DEM close to the epidermis EPM, the effective ingredient EM may be included in the second layer 122.

In another embodiment, although not illustrated in the drawings, the same effective ingredient EM may be included in each of the first layer 121 and the second layer 122. In order to deliver a drug to a portion with a wide range of depth, the same drug may be included in the first layer 121 and the second layer 122.

In another embodiment, although not illustrated in the drawings, the first layer 121 and the second layer 122 may include different effective ingredients EM. For example, a first effective ingredient EM1 included the first layer 121 may be a drug targeted to the dermis DEM, and a second effective ingredient EM2 included in the second layer 122 may be a drug targeted to the epidermis EPM. In this case, the delivery rates of the first effective ingredient EM1 and the second effective ingredient EM2 may be adjusted by adjusting the biodegradation rates of the first layer 121 and the second layer 122.

The first layer 121 and the second layer 122 may have different biodegradation rates after insertion into the skin. Any one of the first layer 121 and the second layer 122 may have a biodegradation rate greater than that of another. The biodegradation rates of the first layer 121 and the second layer 122 may depend on the types and amounts of the biocompatible materials constituting the layers.

For example, when the biodegradation rate of the first layer 121 is greater than the biodegradation rate of the second layer 122, the effective ingredient EM may be rapidly delivered to the dermis DEM. When the biodegradation rate of the second layer 122 is greater than the biodegradation rate of the first layer 121, the effective ingredient EM may be rapidly delivered to the epidermis EPM. In addition, when the biodegradation rate of the second layer 122 is greater than the biodegradation rate of the first layer 121, the second layer 122 may rapidly biodegrade, thus the base 110 may be rapidly removed, and the effective ingredient EM included in the first layer 121 may be released into the skin.

The layers of the microneedle 120 may have different stiffnesses. The first layer 121 and the second layer 122 of the microneedle 120 may have different stiffnesses. For example, when the first layer 121 has a stiffness greater than that of the second layer 122, the first layer 121 may easily penetrate the skin. Then, the first layer 121 may rapidly biodegrade, and thus the pain in the skin may be minimized.

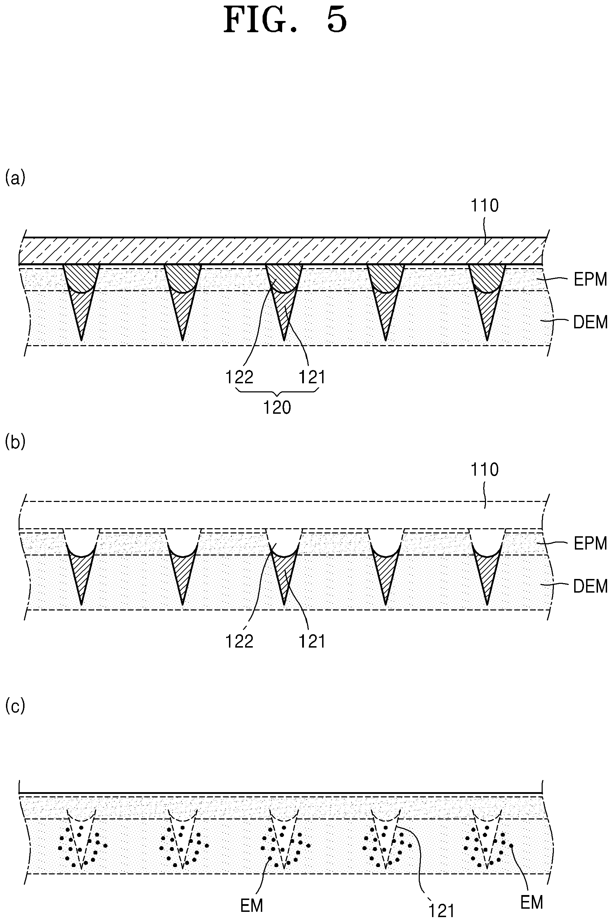

FIG. 5 is a diagram illustrating a process in which the microneedle patch 100 of FIG. 2 is placed on a skin, and then a drug is delivered.

Referring to FIG. 5, the microneedle patch 100 may be placed on the skin, the layers of the microneedle 120 may then biodegrade, and thus the drug may be delivered. Although FIG. 5 illustrates that the effective ingredient EM is included in the first layer 121 and then delivered to the dermis DEM, the effective ingredient EM may be included in the second layer 122 and then delivered to the epidermis EPM.

Referring to (a) of FIG. 5, the microneedle patch 100 is placed on the skin. The microneedle 120 is inserted into the skin, and then the base 110 covers the top of the skin.

Referring to (b) of FIG. 5, the microneedle 120 biodegrades within the skin. When the second layer 122 biodegrades first, the base 110 may be easily separated from the second layer 122.

Referring to (c) of FIG. 5, the effective ingredient EM may be released from the microneedle 120. When the first layer 121 begins to biodegrade, the effective ingredient EM included therein may be delivered to the dermis DEM.

The microneedle patch 100 according to the present disclosure has a multi-layer structure, and thus may accurately deliver the effective ingredient EM to a target point. Because the microneedle 120 includes the plurality of layers, the effective ingredient EM may be included in each layer. Accordingly, the microneedle patch 100 may deliver the effective ingredient EM to any one of the epidermis EPM, the dermis DEM, subcutaneous fat, and muscle, according to a position at which the effective ingredient EM is activated.

Because the microneedle patch 100 according to the present disclosure has the multi-layer structure, the biodegradation rates of the layers may be different from each other. The effective ingredients EM of the layers of the microneedle 120 may be activated at different points of time according to the decomposition rates of the layers.

In the microneedle patch 100 according to the present disclosure, each layer has a curvature, and thus may easily decompose in vivo. As the first layer 121 and the second layer 122 have the curvatures, the distance between a region at which the first layer 121 and the second layer 122 are in contact with each other and the edge of the microneedle 120 is short, and thus the first layer 121 and the second layer 122 may be easily separated from each other.

In the microneedle patch 100 according to the present disclosure, because each layer has a curvature, the surface area of each layer is increased, and accordingly, the delivery effectiveness of the effective ingredient EM may be increased. The first curved surface CS1 of the first layer 121 increases the upper surface area of the first layer 121, and the second curved surface CS2 of the second layer 122 increases the lower surface area of the second layer 122. As the surface areas of the first layer 121 and the second layer 122 are increased, an area through which a drug is delivered may be also increased, and thus, the drug delivery effectiveness may be improved.

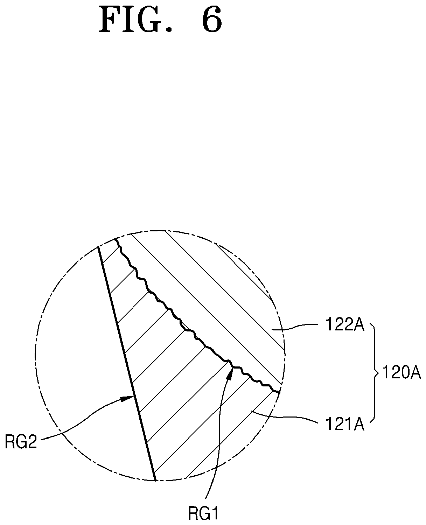

FIG. 6 to FIG. 10 are diagrams illustrating modifications of the microneedle 120 of FIG. 3.

Referring to FIG. 6, in a microneedle 120A, a contact region between adjacent layers may have a preset roughness. The roughness of the contact region between the adjacent layers of the microneedle 120A may be different from the roughness of the outer surface OS of the microneedle 120A.

A contact region between a first layer 121A and a second layer 122A may have a first roughness RG1. A portion where the first curved surface CS1 and the second curved surface CS2 are in contact with each other may have a slightly rough surface.

The outer surface of the microneedle 120A is formed to be smooth. The outer surface of the microneedle 120A may have a second roughness RG2, which may be less than the first roughness RG1.

In a process of manufacturing the microneedle 120A by using a mold, a material of the first layer 121A is first injected into the mold and then dried to manufacture the first layer 121A. Thereafter, a material of the second layer 122A is injected onto the first layer 121A and then dried again to manufacture the second layer 122A.

The outer surface OS of the first layer 121A is formed to be smooth by the surface of the mold, whereas the first curved surface CS1 of the first layer 121A is exposed to the outside during the drying process and thus is formed to be rough. As the second layer 122A is then formed on the first layer 121A, the first curved surface CS1 and the second curved surface CS2 have the first roughness RG1. Because the first roughness RG1 of the contact region between the first layer 121A and the second layer 122A is greater than the second roughness RG2 of the outer surface of the microneedle 120A, the durability and stiffness of the microneedle patch may be enhanced.

The first layer 121A and the second layer 122A are densely formed by the first roughness RG1 of the first layer 121A and the second layer 122A. In order to insert the microneedles 120A into the skin, it is necessary that the first layer 121A and the second layer 122A have a certain stiffness, and the first layer 121A and the second layer 122A not be separated during the insertion of the microneedle 120A. Because each layer of the microneedle 120A is densely formed, the microneedle patch may be safely inserted into the skin.

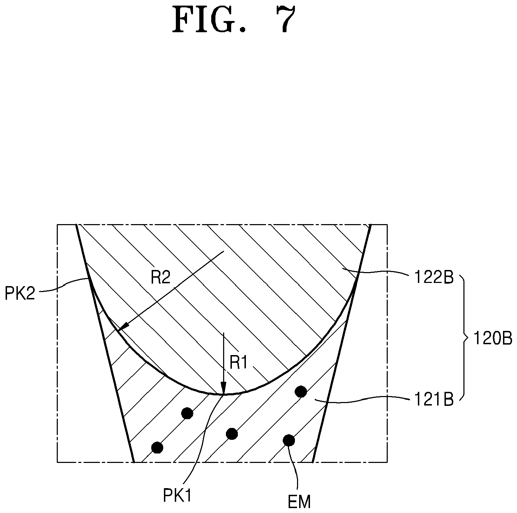

Referring to FIG. 7, a microneedle 120B may include a first layer 121B and a second layer 122B, and may have a plurality of curvatures in a contact region between the first layer 121B and the second layer 122B. At least one of the first curved surface CS1 of the first layer 121B and the second curved surface CS2 of the second layer 122B may have a plurality of curvatures.

The first curved surface CS1 of the first layer 121B may be downwardly convex, and may have a plurality of curvatures.

In detail, the first layer 121B may have a first curvature R1 at the first point PK1, which is on the central axis in the longitudinal direction of the microneedle 120B, and may have a second curvature R2, which is different from the first curvature R1, at an outer side thereof. The first curvature R1 may be greater than the second curvature R2. That is, the radius of curvature of the first curved surface CS1 may be low at the center of the microneedle 120B in the longitudinal direction, and the radius of curvature of the first curved surface CS1 may increase toward the outer side.

In the case where a material, which has a high viscosity and greatly shrinks when dried, is used as the base material of the first layer 121B, the first layer 121B may greatly shrink in a drying process in a state of being strongly attached to the surface of the mold due to the viscosity of the base material.

Because the curvature at the first point PK1, which is on the central axis of the microneedle 120B, is smaller than the curvature at the second point PK2, the first layer 121B and the second layer 122B may be inserted to a deep position. The first layer 121B may include a drug with a high concentration per unit volume, and the drug may be delivered to a deep position. In addition, when the effective ingredient EM is included in the second layer 122B, the effective ingredient EM may reach a deep position.

Furthermore, in the outer side of the microneedle 120B, the thickness of the first layer 121B in the vicinity of the second point PK2 may be thin, thus the first layer 121B and the second layer 122B may be easily separated from each other, and accordingly, the drug delivery effectiveness may be increased.

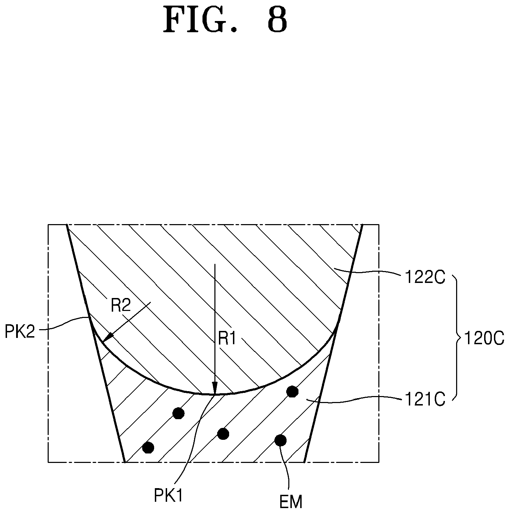

Referring to FIG. 8, a microneedle 120C may include a first layer 121C and a second layer 122C, and may have a plurality of curvatures in a contact region between the first layer 121C and the second layer 122C. At least one of the first curved surface CS1 of the first layer 121C and the second curved surface CS2 of the second layer 122C may have a plurality of curvatures.

The first curved surface CS1 of the first layer 121C may be downwardly convex, and may have a plurality of curvatures.

In detail, the first layer 121C may have the first curvature R1 at the first point PK1, which is on the central axis in the longitudinal direction of the microneedle 120C, and may have the second curvature R2, which is different from the first curvature R1, at an outer side thereof. The first curvature R1 may be greater than the second curvature R2. That is, the radius of curvature of the first curved surface CS1 may be high at the center of the microneedle 120C in the longitudinal direction, and the radius of curvature of the first curved surface CS1 may decrease toward the outer side.

In the case where a material, which has a low viscosity and relatively less shrinks when dried, is used as the base material of the first layer 121C, the difference between the first point PK1 and the second point PK2 may be smaller than that of the embodiment of FIG. 7.

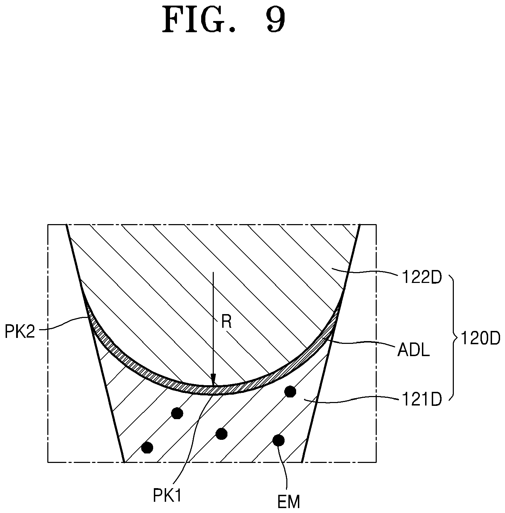

Referring to FIG. 9, a microneedle 120D may include a first layer 121D and a second layer 122D, and an adhesive layer ADL may be arranged between the first layer 121D and the second layer 122D.

The microneedle 120D may be manufactured by forming the first layer 121D, then adding an adhesive material to form the adhesive layer ADL, and forming the second layer 122D on the adhesive layer ADL.

In an embodiment, the adhesive layer ADL may be formed of a material capable of increasing an adhesive force between the first layer 121D and the second layer 122D. In addition, the adhesive layer ADL may be formed of a biocompatible material or a biodegradable material. For example, the adhesive layer ADL may be formed as a moisture layer.

In an embodiment, the adhesive layer ADL may be formed of a material having a biodegradation rate greater than those of the first layer 121D and the second layer 122D. When the microneedle 120D is inserted into the skin, the adhesive layer ADL decomposes first, and thus the first layer 121D and the second layer 122D may be separated from each other.

Although FIG. 9 illustrates that the adhesive layer ADL is distinguished from the first layer 121D and the second layer 122D, the present disclosure is not limited thereto. For example, the adhesive layer ADL may be mixed with the first layer 121D and the second layer 122D. The adhesive layer ADL may be formed by mixing that the base material of the first layer 121D with the base material of the second layer 122D.

Because the microneedle 120D includes the adhesive layer ADL, the coupling strength between the first layer 121D and the second layer 122D may be increased. When the microneedle 120D is being inserted into the skin, the first layer 121D and the second layer 122D are not easily separated from each other due to an external force applied to the microneedle patch. After the microneedle 120D is inserted into the skin, the adhesive layer ADL may easily decompose to separate the first layer 121D and the second layer 122D from each other.

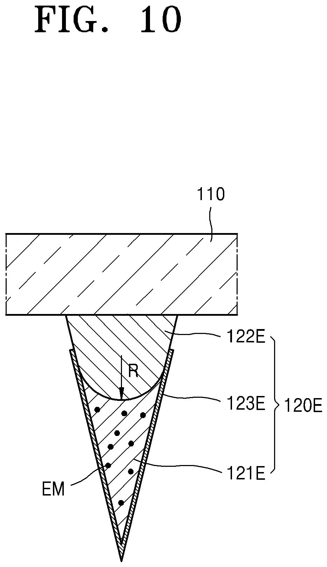

Referring to FIG. 10, a microneedle 120E may include a first layer 121E and a second layer 122E, and a coating layer 123E may be arranged on the outer surface of the microneedle 120E.

The coating layer 123E may be formed by forming the first layer 121E and the second layer 122E and then dipping the first layer 121E and the second layer 122E into a coating solution.

The coating layer 123E may be formed of a biocompatible polymer. The coating layer 123E may decompose when inserted into the skin.

In an embodiment, the coating layer 123E may include a physiologically active substance. When the coating layer 123E is inserted into the skin, the coating layer 123E may become activated first before the effective ingredient EM is injected, and thus, the delivery effectiveness the effective ingredient EM may be increased.

In an embodiment, the coating layer 123E may be formed of a material having a high biodegradation rate. The coating layer 123E may be formed of a material having a biodegradation rate greater than that of the first layer 121E or the second layer 122E, and thus, the in vivo decomposition rate of the coating layer 123E may be greater than the in vivo decomposition rate of the first layer 121E or the in vivo decomposition rate of the second layer 122E.

In another embodiment, the coating layer 123E may be formed of a material having a low biodegradation rate. The coating layer 123E may be formed of a material having a lower biodegradation rate than that of the first layer 121E or the second layer 122E, and thus, the in vivo decomposition rate of the coating layer 123E may be lower than the in vivo decomposition rate of the first layer 121E or the in vivo decomposition rate of the second layer 122E. After the microneedle 120E is inserted into the skin, a drug may be delivered after a certain period of time has elapsed, and thus the effective ingredient EM may be delivered at a preferred appropriate point of time.

In an embodiment, the coating layer 123E may increase the stiffness of the microneedle 120E. Because the coating layer 123E covers an outer side of the boundary between the first layer 121E and the second layer 122E, the first layer 121E and the second layer 122E may be prevented from being separated from each other when the microneedle 120E is inserted into the skin.

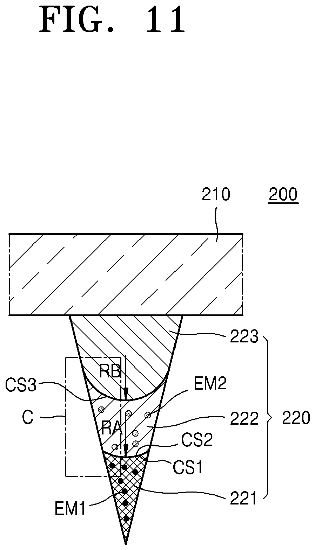

FIG. 11 is a diagram illustrating a microneedle patch 200 according to another embodiment of the present disclosure.

Referring to FIG. 11, the microneedle patch 200 may include a base 210 and a microneedle 220, and the microneedle 220 may include a plurality of layers. The microneedle 220 may include a first layer 221, a second layer 222, and a third layer 223.

Each layer of the microneedle 220 may be formed of a biocompatible material and an additive.

The microneedle 220 may include the effective ingredient EM. Because the microneedle 220 has a structure in which the plurality of layers are stacked, a plurality of effective ingredients may be arranged in the plurality of layers, respectively.

Although FIG. 11 illustrates that the first effective ingredient EM1 is arranged in the first layer 221 and the second effective ingredient EM2 is arranged in the second layer 222, the present disclosure is not limited thereto. For example, an effective ingredient may be arranged in only one of the first layer 221 to the third layer 223, or effective ingredients may be arranged in two layers selected from the first layer 221 to the third layer 223. In addition, effective ingredients may be arranged in all of the first layer 221 to the third layer 223. The effective ingredients in the plurality of layers may be the same as or different from each other.

The microneedle 220 may include the first layer 221 arranged at the tip thereof, the second layer 222 stacked on the first layer 221, and the third layer 223 stacked on the second layer 222. The microneedle 220 may have a curvature in a region where adjacent layers are in contact with each other.

A curvature may be formed in a region where the first layer 221 and the second layer 222 are in contact with each other, or a region where the second layer 222 and the third layer 223 are in contact with each other. The microneedle 220 may have a curvature downwardly convex toward the tip. The region where the first layer 221 and the second layer 222 are in contact with each other, or the region where the second layer 222 and the third layer 223 are in contact with each other may be downwardly convex toward the tip.

The first layer 221 may have the sharpened tip ST arranged at one side thereof, and the first curved surface CS1 depressed toward the sharpened tip ST at the other side thereof.

The second layer 222 may face the first curved surface CS1, and may have the second curved surface CS2 protruding to correspond to the first curved surface CS1. In addition, the second layer 222 may have a third curved surface CS3 arranged at the opposite side to the second curved surface CS2 and recessed toward the second curved surface CS2. The third curved surface CS3 may be in contact with the third layer 223.

In an embodiment, a first curvature RA formed between the first layer 221 and the second layer 222 may be substantially the same as a second curvature RB formed between the second layer 222 and the third layer 223.

The second curved surface CS2 may be connected to the first curved surface CS1, and thus, the first curved surface CS1 and the second curved surface CS2 may form a contact region. Accordingly, the curvature of the second curved surface CS2 may be substantially the same as the curvature of the first curved surface CS1. In addition, the curvature of the second curved surface CS2 may be substantially the same as the curvature of the third curved surface CS3. Alternatively, the curvature of the second curved surface CS2 and the curvature of the third curved surface CS3 may be substantially the same in some sections, and may be differently from each other in other sections.

Like the microneedle patch 100 according to the above-described embodiment, the decomposition rates of the layers of the microneedle 220 may be different from each other. According to the depth to which the microneedle 220 is inserted, the effective ingredients EM are arranged in the respective layers, and, because the layers have different in vivo decomposition rates, the effective ingredients EM may be appropriately delivered at desired points of time, respectively.

For example, in the case where the second effective ingredient EM2 is to be released after the first effective ingredient EM1 is released in order to enhance the drug effectiveness of the first effective ingredient EM1, the layer including the first effective ingredient EM1 may be decomposed first, and then the layer including the second effective ingredient EM2 may be decomposed.

Because the microneedle 220 has a layered structure with curvatures, each layer may be easily separated. The distance between a portion at which the first layer 221 and the second layer 222 are in contact with each other and the edge of the microneedle 220 is short.

In detail, the distance between the outer surface OS of the first layer 221 and the first curved surface CS1 is relatively short. In addition, the distance between the outer surface OS of the second layer 222 and the third curved surface CS3 is relatively short. When the microneedle 220 is inserted into the skin, the outer surface OS of the microneedle 220 begins to biodegrade, and because the first layer 221 is easily separated by the first curved surface CS1, the first layer 221 and the second layer 222 may be easily separated from each other. In addition, because the second layer 222 is easily separated by the third curved surface CS3, the second layer 222 and the third layer 223 may be easily separated from each other.

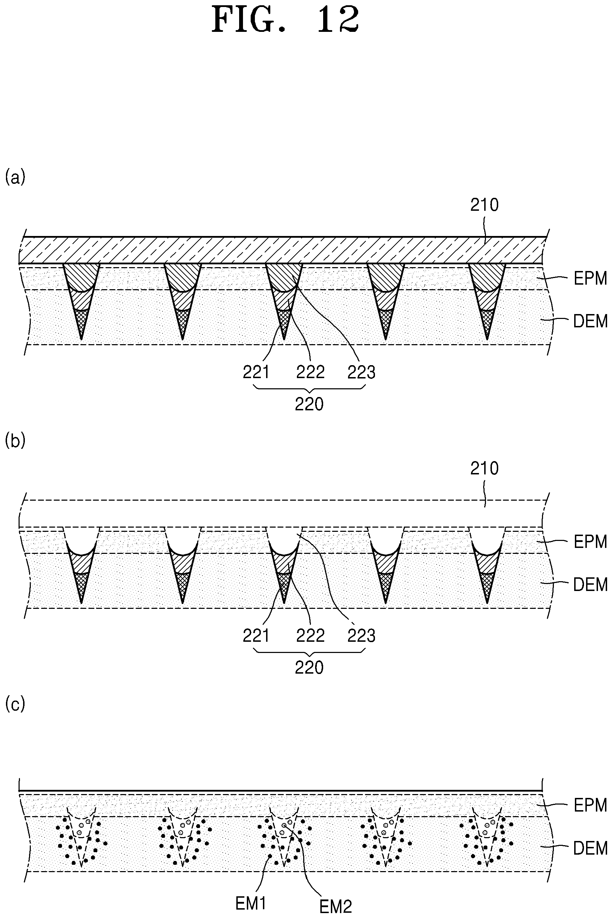

FIG. 12 is a diagram illustrating a process in which the microneedle patch 200 of FIG. 11 is placed on a skin, and then a drug is delivered.

Referring to FIG. 12, the first effective ingredient EM1 may be included in the first layer 221, the second effective ingredient EM2 may be included in the second layer 222, and positions to which the effective ingredients are to be delivered may depend on the positions of the effective ingredients.

Referring to (a) of FIG. 12, the microneedle patch 200 is placed on the skin. The microneedle 220 is inserted into the skin, and then the base 210 covers the top of the skin.

Referring to (b) of FIG. 12, the microneedle 220 biodegrades within the skin. When the third layer 223 biodegrades first, the base 210 may be easily separated from the third layer 223.

Referring to (c) of FIG. 12, the effective ingredients EM1 and EM2 may be released from the microneedle 220. When the first layer 221 begins to biodegrade, the first effective ingredient EM1 included therein may be delivered to the dermis DEM, and when the second layer 222 begins to biodegrade, the second effective ingredient EM2 included therein may be delivered to the dermis DEM. In this case, the first effective ingredient EM1 and the second effective ingredient EM2 may interact with each other to enhance the pharmacological effect in the dermis DEM.

Although FIG. 12 illustrates an example in which all of the effective ingredients are delivered to the dermis, but the present disclosure is not limited thereto. For example, the effective ingredients may be delivered to only the epidermis, or both the epidermis and the dermis.

The microneedle patch 200 according to the present disclosure has a multi-layer structure, and thus may accurately deliver the effective ingredients EM1 and EM2 to a target point. Because the microneedle 220 include a plurality of layers, the effective ingredients EM1 and EM2 may be included in the respective layers. Accordingly, the microneedle patch 200 may deliver the effective ingredients EM1 and EM2 to any one of the epidermis EPM, the dermis DEM, subcutaneous fat, and muscle, according to positions at which the effective ingredients EM1 and EM2 are activated.

Because the microneedle patch 200 according to the present disclosure has a multi-layer structure, the biodegradation rates of the layers may be different from each other. The effective ingredients EM1 and EM2 of the layers of the microneedle 220 may be activated at different points of time according to the decomposition rates of the layers.

In the microneedle patch 200 according to the present disclosure, each layer has a curvature, and thus may easily decompose in vivo. In the case where the first layer 221 and the second layer 222 have curvatures, the contact region of the first layer 221 and the second layer 222 is thin at the edge of the microneedle 220 is short, and thus the first layer 221 and the second layer 222 may be easily separated from each other. In addition, as the second layer 222 and the third layer 223 have the curvatures, the distance between a region at which the second layer 222 and the third layer 223 are in contact with each other and the edge of the microneedle 220 is short, and thus the second layer 222 and the third layer 223 may be easily separated from each other.

In the microneedle patch 200 according to the present disclosure, because each layer has a curvature, the surface area of each layer is increased, and accordingly, the delivery effectiveness of the effective ingredients EM1 and EM2 may be increased. The first curved surface CS1 of the first layer 221 increases the upper surface area of the first layer 221, and the second curved surface CS2 of the second layer 222 increases the lower surface area of the second layer 222. In addition, the third curved surface CS3 of the second layer 222 increases the upper surface area of the second layer 222 and the lower surface area of the third layer 223. As the surface areas of the first layer 221, the second layer 222, and the third layer 223 are increased, an area through which a drug is delivered may be also increased, and thus, the drug delivery effectiveness may be improved.

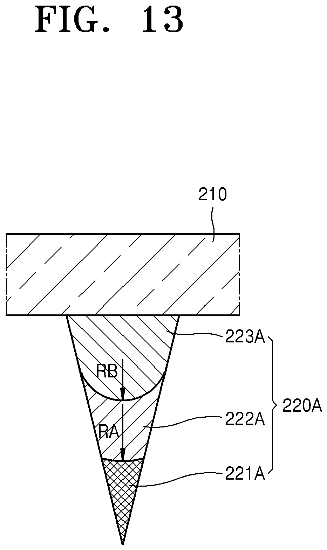

FIG. 13 to FIG. 18 are diagrams illustrating modifications of the microneedle patch 200 of FIG. 11.

Referring to FIG. 13, a microneedle 220A includes a first layer 221A, a second layer 222A, and a third layer 223A.

The first curvature RA formed between the first layer 221A and the second layer 222A may be different from the second curvature RB formed between the second layer 222A and the third layer 223A. The first curvature RA formed between the first layer 221A and the second layer 222A may be smaller than the second curvature RB formed between the second layer 222A and the third layer 223A.

The base material of the first layer 221A may have an adhesive force less than that of the base material of the second layer 222A, and may less shrink when dried than does the base material of the second layer 222A. The base material of the first layer 221A is injected into a mold and then dried, and then the base material of the second layer 222A is injected into the mold, and then dried. Because the base material of the second layer 222A has a greater adhesive force and more shrinks when dried than the base material of the first layer 221A, the second layer 222A may have a larger curvature.

The second layer 222A is formed to have a thin upper edge due to a large curvature of an upper portion thereof. When the microneedle 220A is inserted into the skin, the edge of the second layer 222A may rapidly decompose, and thus the second layer 222A and the third layer 223A may be easily separated from each other.

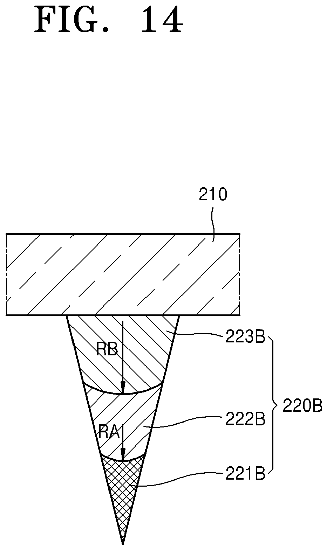

Referring to FIG. 14, a microneedle 220B includes a first layer 221B, a second layer 222B, and a third layer 223B.

The first curvature RA formed between the first layer 221B and the second layer 222B may be different from the second curvature RB formed between the second layer 222B and the third layer 223B. The first curvature RA formed between the first layer 221B and the second layer 222B may be larger than the second curvature RB formed between the second layer 222B and the third layer 223B.

The base material of the first layer 221B may have an adhesive force greater than that of the base material of the second layer 222B, and may more shrink when dried than does the base material of the second layer 222B. The base material of the first layer 221B is injected into a mold and then dried, and then the base material of the second layer 222B is injected into the mold, and then dried. Because the base material of the first layer 221B has a greater adhesive force and more shrinks when dried than the base material of the second layer 222B, the first layer 221B may have a larger curvature.

The first layer 221B is formed to have a thin upper edge due to a large curvature of an upper portion thereof. When the microneedle 220B is inserted into the skin, the edge of the first layer 221B may rapidly decompose, and thus the first layer 221B and the second layer 222B may be easily separated from each other.

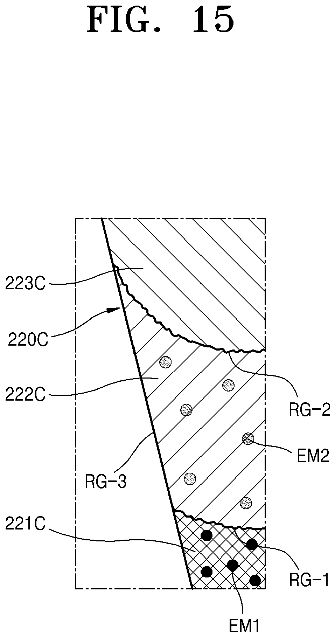

Referring to FIG. 15, in a microneedle 220C, a contact region between adjacent layers may have a preset roughness. The roughness of the contact region between the adjacent layers of the microneedle 220C may be different from the roughness of the outer surface OS of the microneedle 220C.

A contact region between a first layer 221C and a second layer 222C may have a first roughness RG-1. The contact region between the first layer 221C and the second layer 222C may have a slightly rough surface.

A contact region between the second layer 222C and a third layer 223C may have a second roughness RG-2. The contact region between the second layer 222C and the third layer 223C may have a slightly rough surface.

The outer surface of the microneedle 220C is formed to be smooth. The outer surface of the microneedle 220C may have a third roughness RG-3, which may be less than the first roughness RG-1 or the second roughness RG-2.

The outer surface OS of the microneedle 220C is formed to be smooth by the surface of the mold, whereas the first curved surface CS1 of the first layer 221C is exposed to the outside during the drying process and thus is formed to be rough. Thereafter, as the second layer 222C is formed on the first layer 221C, the microneedle 220C has the first roughness RG-1 between the first layer 221C and the second layer 222C. In addition, the third curved surface CS3 of the second layer 222C is exposed to the outside during the drying process, and thus is formed to be rough. Thereafter, as the third layer 223C is formed on the second layer 222C, the microneedle 220C has the second roughness RG-2 between the second layer 222C and the third layer 223C.

The first roughness RG-1 of the contact region between the first layer 221C and the second layer 222C and/or the second roughness RG-2 of the contact region between the second layer 222C and the third layer 223C is greater than the third roughness RG-3 of the outer surface of the microneedle 220C, and thus the durability and stiffness of the microneedle patch may be enhanced.

The first roughness RG-1 causes the first layer 221C and the second layer 222C to be dense, and the second roughness RG-2 causes the second layer 222C and the third layer 223C to be dense. When the microneedle 220C is being inserted into the skin, the layers may not be separated from each other. Because each layer of the microneedle 220C is formed to be dense, the microneedle patch may be safely inserted into the skin.

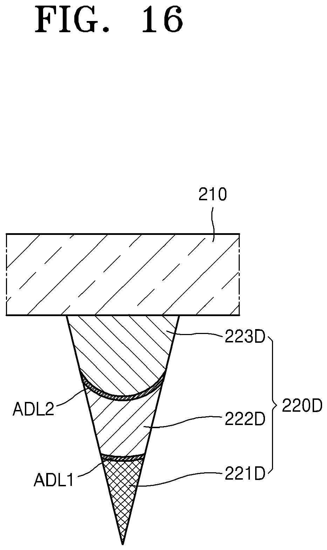

Referring to FIG. 16, in a microneedle 220D, a first adhesive layer ADL1 may be arranged between a first layer 221D and a second layer 222D, and a second adhesive layer ADL2 may be arranged between the second layer 222D and a third layer 223D.

To form the microneedle 220D, the first layer 221D may be formed, then an adhesive material may be added onto the first layer 221D form the first adhesive layer ADL1, and the second layer 222D may be formed on the first adhesive layer ADL1. Thereafter, an adhesive material may be added onto the second layer 222D to form the second adhesive layer ADL2, and the third layer 223D may be formed on the second adhesive layer ADL2 to finally manufacture the microneedle 220D.

In an embodiment, the first adhesive layer ADL1 and the second adhesive layer ADL2 may be formed of a material capable of increasing an adhesive force between layers adjacent to each other. In addition, the first adhesive layer ADL1 and the second adhesive layer ADL2 may be formed of a biocompatible material or a biodegradable material. For example, the first adhesive layer ADL1 and the second adhesive layer ADL2 may be formed as a moisture layer.

In an embodiment, the first adhesive layer ADL1 and the second adhesive layer ADL2 may be formed of a material having a biodegradation rate greater than those of the first layer 221D, the second layer 222D, and the third layer 223D. When the microneedle 220D is inserted into the skin, the first and second adhesive layers ADL1 and ADL 2 decompose first, and thus the first layer 221D and the second layer 222D or the second layer 222D and the third layer 223D may be separated from each other.

Although FIG. 16 illustrates that the first adhesive layer ADL1 is distinguished from the first layer 221D and the second layer 222D, the present disclosure is not limited thereto. For example, the first adhesive layer ADL1 may be mixed with the first layer 221D and the second layer 222D. The first adhesive layer ADL1 may be formed by mixing that the base material of the first layer 221D with the base material of the second layer 222D. The second adhesive layer ADL2 may be formed in the same manner as the first adhesive layer ADL1.

Because the microneedle 220D includes the adhesive layers, the coupling strength between the stacked layers may be increased. When the microneedle 220D is being inserted into the skin, the layers are not easily separated from each other due to an external force applied to the microneedle patch. After the microneedle 220D is inserted into the skin, the adhesive layers may easily decompose, and thus the first layer 221D and the second layer 222D or the second layer 222D and the third layer 223D may be separated from each other.

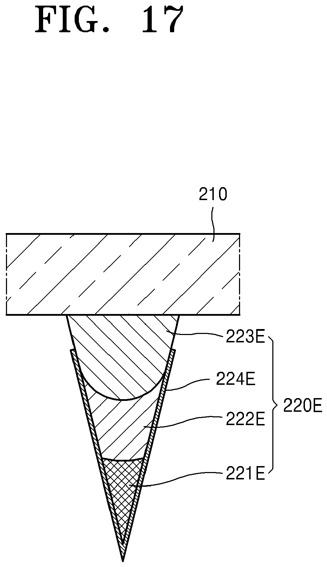

Referring to FIG. 17, a microneedle 220E may include a first layer 221E, a second layer 222E, and a third layer 223E, and a coating layer 224E may be arranged on the outer surface of the microneedle 220E.

The coating layer 224E may be formed by forming the first layer 221E, the second layer 222E, and the third layer 223E and then dipping the first layer 221E, the second layer 222E, and the third layer 223E into a coating solution.

The coating layer 224E may be formed of a biocompatible material. The coating layer 224E may decompose when inserted into the skin.

In an embodiment, the coating layer 224E may include a physiologically active substance. When the coating layer 224E is inserted into the skin, the coating layer 224E may be activated first before the effective ingredient EM is injected, and thus, the delivery effectiveness the effective ingredient EM may be increased.

In an embodiment, the coating layer 224E may be formed of a material having a high biodegradation rate. The coating layer 224E may be formed of a material having a biodegradation rate higher than that of any one of the first layer 221E, the second layer 222E, and the third layer 223E.

In another embodiment, the coating layer 224E may be formed of a material having a low biodegradation rate. The coating layer 224E may be formed of a material having a biodegradation rate less than that of any one of the first layer 221E, the second layer 222E, and the third layer 223E, and thus the in vivo decomposition rate of the coating layer 224E may be less than that of the first layer 221E, the second layer 222E, or the third layer 223E. After the microneedle 220E is inserted into the skin, a drug may be delivered after a certain period of time has elapsed, and thus the effective ingredient may be delivered at a preferred appropriate point of time.

In an embodiment, the coating layer 224E may increase the stiffness of the microneedle 220E. Because the coating layer 224E covers an outer side of the boundary between the first layer 221E and the second layer 222E or covers the boundary between the second layer 222E and the third layer 223E, the layers may be prevented from being separated from each other when the microneedle 220E is inserted into the skin.

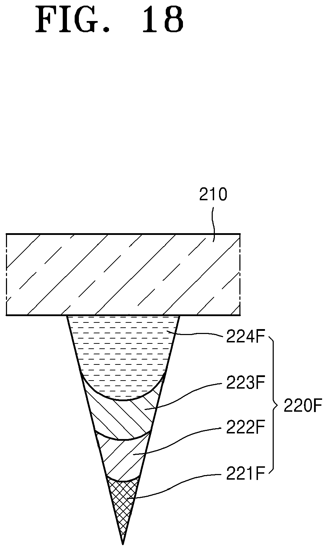

Referring to FIG. 18, a microneedle patch may include the base 210 and a microneedle 220F, and the microneedle 220F may include a first layer 221F, a second layer 222F, a third layer 223F, and a fourth layer 224F. The layered structure of the layers may be the same as that in the above-described embodiments.

The microneedle 220F may have a plurality of layers, and at least one of the layers may have a curvature.

Although FIG. 18 illustrates four layers, the present disclosure is not limited thereto, and the microneedle 220F may have five or more layers.

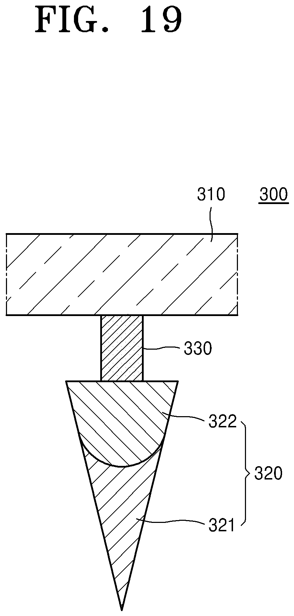

FIG. 19 is a diagram illustrating a microneedle patch 300 according to another embodiment of the present disclosure.

Referring to FIG. 19, the microneedle patch 300 may include a base 310, a microneedle 320, and a shaft 330. The microneedle 320 may have a layered structure of a first layer 321 and a second layer 322, and may include a curvature in the boundary region between the first layer 321 and the second layer 322.

The microneedle 320 may be the same as the microneedle 120 described above. As described above, the microneedle 320 may precisely deliver an effective ingredient to a target position.

The shaft 330 may connect the base 310 to the microneedle 320. The shaft 330 may extend a certain distance in the longitudinal direction of the microneedle 320. The shaft 330 may allow the microneedle 320 to be deeply inserted. That is, the length of the shaft 330 may allow the effective ingredient EM of the microneedle patch 300 to be deeply delivered to a deep position under the skin of a patient.

The shaft 330 may decompose in vivo to be easily separated from the base 310. Because the volume of the shaft 330 is smaller than that of the microneedle 320, the shaft 330 may be dissolved in vivo earlier than is the microneedle 320. After the shaft 330 is dissolved, the microneedle 320 is within the skin of the patient, and the base 310 may be easily removed.

The shaft 330 may decompose in vivo faster than the microneedle 320. The base material of the shaft 330 may be formed of a material that decomposes in vivo faster than the microneedle 320. Thus, when the microneedle patch 300 is inserted into the skin of the patient, the shaft 330 may be rapidly dissolved, and the base 310 may be easily removed.

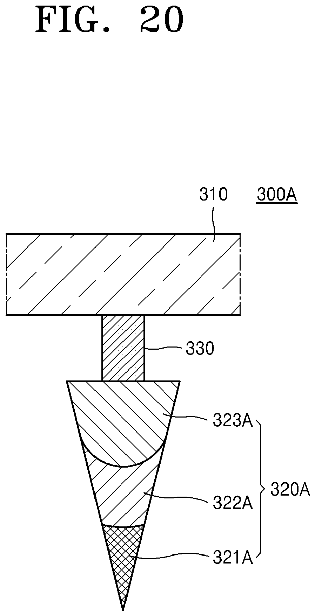

FIG. 20 is a diagram illustrating a modification of the microneedle patch 300 of FIG. 19.

Referring to FIG. 20, a microneedle patch 300A may include the base 310, the microneedle 320, and the shaft 330. The microneedle 320A may have a layered structure of a first layer 321A, a second layer 322A, and a third layer 323A, and may include curvatures in the boundary regions between the first layer 321A and the second layer 322A, and between the second layer 322A and the first layer 323A, respectively.

The microneedle 320A may be the same as the microneedle 220 described above. As described above, the microneedle 320A may precisely deliver an effective ingredient to a target position. In addition, the shaft 330 may allow the effective ingredient EM to be delivered to a deep position, and the base 310 may be easily removed as the shaft 330 is dissolved.

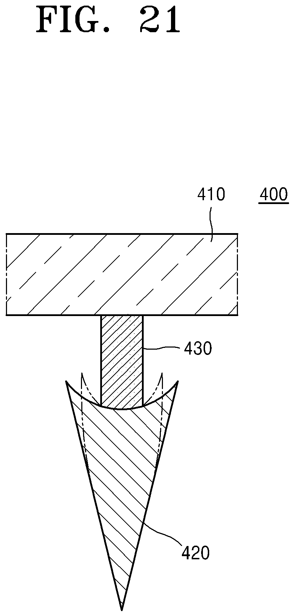

FIG. 21 is a diagram illustrating a microneedle patch 400 according to another embodiment of the present disclosure.

Referring to FIG. 21, the microneedle patch 400 may include a base 410, a microneedle 420, and a shaft 430.

The upper surface of the microneedle 420 may have a curvature. A surface of the microneedle 420, which is in contact with the shaft 430, may have a downwardly convex curvature. Because the upper surface of the microneedle 420 is formed to be downwardly convex, the microneedle 420 has a thin edge at the upper end thereof. As the edge of the upper end of the microneedle 420 is formed to be thin, the edge of the microneedle 420 may have a certain flexibility.

The flexible edge of the microneedle 420 may allow the microneedle 420 to be easily inserted into the skin of the patient and to remain inserted in the skin.

As illustrated in FIG. 21, when the microneedle patch 400 is inserted into the skin, the upper edge of the microneedle 420 is compressed toward the shaft 430 by an external pressure. The microneedle 420 is compressed in the longitudinal direction, and thus may be easily inserted into the skin.

After inserted into the skin, the upper edge of the microneedle 420 may expand outward, and then be supported under the skin. The position of the microneedle 420 is fixed under the skin of the patient, and the upper edge of the microneedle 420 allows the microneedle 420 not to be pulled out of the skin even when the base 410 is removed.

The microneedle patch 400 according to the present disclosure may be easily inserted into the skin of the patient, and may be fixed in position by the curvature of the upper surface thereof. Because the upper edge of the microneedle 420 is flexible, the microneedle 420 may be easily inserted into the skin and stably maintained in position after being inserted.

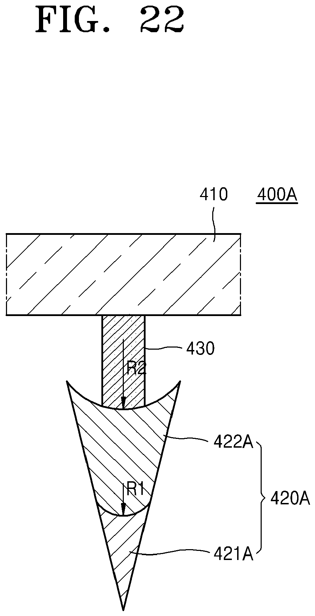

FIG. 22 is a diagram illustrating a modification of the microneedle patch 400 of FIG. 21.

Referring to FIG. 22, a microneedle patch 400A may include the base 410, a microneedle 420A, and the shaft 430. The microneedle 420A may include a first layer 421A and a second layer 422A.

The first curvature R1 may be formed in the boundary region between the first layer 421A and the second layer 422A. The structure between the first layer 421A and the second layer 422A may be the same as the structure of the first layer 121 and the second layer 122 described above or a modified structure thereof.

The second curvature R2 may be formed on the upper surface of the second layer 422A. The upper edge of the second layer 422A may be flexible due to the second curvature R2. The flexible edge of the second layer 422A may allow the microneedle 420A to be easily inserted into the skin of the patient and to remain inserted in the skin.

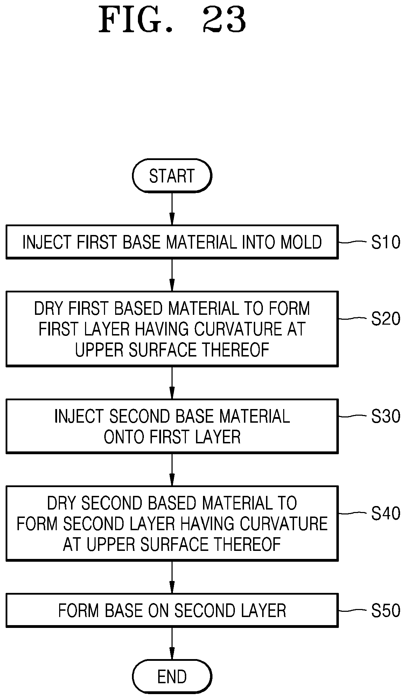

FIG. 23 is a flowchart of a method of manufacturing a microneedle patch, according to another embodiment of the present disclosure.

Referring to FIG. 23, the microneedle patch may be manufactured by injecting a base material into a mold and drying the base material.

The method of manufacturing a microneedle patch may include injecting a first base material into the mold (S10), forming a first layer having a curvature on the upper surface thereof by drying the first base material (S20), injecting a second base material onto the first layer (S30), forming a second layer having a curvature on the upper surface thereof by drying the second base material (S40), and forming a base on the second layer (S50).

In the injecting of the first base material into the mold (S10), the first base material is injected into the mold (not shown) to form the sharpened tip ST of the microneedle 120. The first base material may include a biocompatible polymer or an adhesive. In an alternative embodiment, the first base material may include the effective ingredient EM.

In the drying of the first base material to form the first layer having the curvature on the upper surface thereof (S20), the first base material is dried. At this time, the viscosity and drying-induced shrinkage of the first base material cause the upper surface of the first layer to be downwardly convex.

In the injecting of the second base material onto the first layer (S30), the second base material is injected to form the second layer on the first layer 121. The second base material may include a biocompatible polymer or an adhesive. In an alternative embodiment, the second base material may include the effective ingredient EM.

In an alternative embodiment, an adhesive layer may be formed by adding an adhesive material before injecting the second base material.

In the drying of the second base material to form the second layer having the curvature on the upper surface thereof (S40), the second base material is dried. At this time, the viscosity and drying-induced shrinkage of the second base material cause the upper surface of the second layer to be downwardly convex.

In the forming of the base on the second layer (S50), the base may be formed on the second layer.

For example, the base, which is prepared in a plate shape, may be attached onto the second layer, and a plurality of microneedles may be attached to one surface of the base.

As another example, a third base material constituting the base may be injected onto the second layer. The third base material may be combined with the second layer, such that the plurality of microneedles are arranged on one surface of the base.

In another embodiment, the third base material may be injected into another mold to prepare a base and a shaft. Thereafter, the second layer and the shaft may be aligned with each other, then the shaft may be attached to the second layer, and a plurality of microneedles may be arranged on one surface of the base.

Although the present disclosure has been described with reference to the embodiments illustrated in the drawings, they are merely exemplary, and it will be understood by one of skill in the art that various modifications and equivalent embodiments may be made therefrom. Therefore, the true technical protection scope of the present disclosure should be determined by the appended claims.

The particular implementations shown and described herein are illustrative examples of embodiments and are not intended to otherwise limit the scope of embodiments in any way. Moreover, no item or component is essential to the practice of the present disclosure unless the item or component is specifically described as "essential" or "critical".

The term "the" and other demonstratives similar thereto in the descriptions of embodiments (especially in the following claims) should be understood to include a singular form and plural forms. Furthermore, recitation of ranges of values herein are merely intended to serve as a shorthand method of referring individually to each separate value falling within the range, unless otherwise indicated herein, and each separate value is incorporated into the specification as if it were individually recited herein. Finally, the operations of all methods described herein may be performed in any suitable order unless otherwise indicated herein or otherwise clearly contradicted by context. The present disclosure is not limited to the described order of the operations. The use of any and all examples, or exemplary language (e.g., "and the like") provided herein, is intended merely to better illuminate embodiments and does not pose a limitation on the scope of the embodiments unless otherwise claimed. In addition, various modifications, combinations, and adaptations will be readily apparent to those skilled in this art without departing from the following claims and equivalents thereof.

## Claims

1. A microneedle patch comprising:
a base; and
a microneedle, which protrudes from a surface of the base, comprises a plurality of layers, and has a curvature in a region where the layers are in contact with each other.

2. The microneedle patch of claim 1, wherein
the microneedle comprises:
a first layer having a sharpened tip at one side thereof and a first curved surface at the other side thereof, the first curved surface being recessed toward the sharpened tip; and
a second layer arranged to face the first curved surface and having a second curved surface protruding to correspond to the first curved surface.

3. The microneedle patch of claim 2, wherein
the second layer has a third curved surface arranged to be opposite to the second curved surface and recessed toward the second curved surface.

4. The microneedle patch of claim 3, wherein
the curvature of the first curved surface and a curvature of the third curved surface are different from each other.

5. The microneedle patch of claim 2, wherein
at least one of the first curved surface and the second curved surface has a plurality of curvatures.

6. The microneedle patch of claim 2, wherein
both ends of the curvature of the first curved surface are symmetrical to each other with respect to a longitudinal direction of the microneedle.

7. The microneedle patch of claim 1, wherein
, in the microneedle,
a roughness of a region where adjacent layers are in contact with each other is different from a roughness of an outer surface of the microneedle.

8. The microneedle patch of claim 1, wherein
the microneedle further comprises an adhesive layer arranged between the adjacent layers.

9. The microneedle patch of claim 1, wherein
at least one of the plurality of layers of the microneedle comprises an in vivo degradable polymer.

10. A microneedle patch comprising:
a base; and
a microneedle protruding from a surface of the base and comprising a plurality of layers,
wherein a roughness of a region where adjacent layers are in contact with each other is different from a roughness of an outer surface of the microneedle.
